(19) Europäisches Patentamt
European Patent Office
Office européen des brevets

(11) EP 4 360 634 A1

(12) **EUROPEAN PATENT APPLICATION**
published in accordance with Art. 153(4) EPC

(43) Date of publication:
**01.05.2024 Bulletin 2024/18**

(21) Application number: **22827568.1**

(22) Date of filing: **21.06.2022**

(51) International Patent Classification (IPC):
***A61K 31/4985*** *(2006.01)* ***A61P 35/00*** *(2006.01)*

(52) Cooperative Patent Classification (CPC):
**A61K 31/4985; A61P 35/00**

(86) International application number:
**PCT/CN2022/100134**

(87) International publication number:
**WO 2022/268075 (29.12.2022 Gazette 2022/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.06.2021 PCT/CN2021/101322**

(71) Applicant: **Betta Pharmaceuticals Co., Ltd**
**Yuhang**
**Hangzhou**
**Zhejiang 311100 (CN)**

(72) Inventors:
- **GUO, Jing**
  **Beijing 100176 (CN)**
- **YAN, Dan**
  **Beijing 100176 (CN)**
- **WANG, Yu**
  **Beijing 100176 (CN)**
- **XU, Tong**
  **Beijing 100176 (CN)**
- **LI, Kao**
  **Beijing 100176 (CN)**
- **XU, Wei**
  **Beijing 100176 (CN)**
- **LAN, Hong**
  **Beijing 100176 (CN)**
- **DING, Lieming**
  **Hangzhou, Zhejiang 311100 (CN)**
- **WANG, Jiabing**
  **Beijing 100176 (CN)**

(74) Representative: **Bardehle Pagenberg Partnerschaft mbB**
**Patentanwälte Rechtsanwälte**
**Prinzregentenplatz 7**
**81675 München (DE)**

# (54) APPLICATION OF COMPOUND IN PREPARATION OF DRUG FOR TREATING MYELOFIBROSIS AND RELATED SYMPTOMS/SIGNS THEREOF, AND USE OF COMPOUND

(57) The present invention provides an application of a compound in the preparation of drug for treating myelofibrosis and the related symptoms/signs thereof, and a use of the compound. The compound is selected form one or more of a compound having a structure as shown in formula I, and a pharmaceutically-acceptable salt, prodrug, solvate and hydrate thereof.

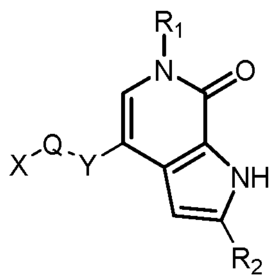

I

EP 4 360 634 A1

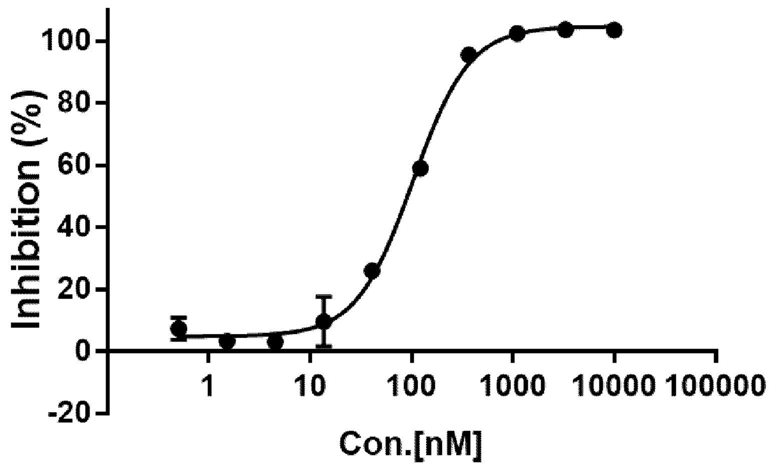
Inhibition (%)
100
80
60
40
20
0
-20
1
10
100
1000
10000
100000
Con.[nM]

Figure 2

## Description

FIELD OF THE INVENTION

The present invention relates to the field of medicament, specifically, relates to a use of a compound in the preparation of the medicament for treating myelofibrosis and the symptoms/signs associated with myelofibrosis, and the use thereof.

BACKGROUND OF THE INVENTION

Myelofibrosis (MF) is a myeloproliferative neoplasm (MPNs) caused by the clonal proliferation of hematopoietic stem cells, including primary myelofibrosis, myelofibrosis secondary to polycythemia vera and essential thrombocythemi. Myelofibrosis (MF) is a disease characterized by the occurrence of reticular fibers and collagen fibers in the bone marrow, the clinical manifestations of MF include anemia, fatigue, early satiety, abdominal imbalance, poor mobility, difficulty concentrating, night sweats, itchy skin, diffuse bone pain, fever, weight loss, splenomegaly, hepatomegaly, which impacts quality of life seriously. The overall median survival time is 3.5 to 5.5 years, but the higher the risk level, the shorter the survival time, the median survival time of high-risk patients is about 2 years. The cause of myelofibrosis (MF) has not been well understood, most patients carry an activating mutation of the Janus kinase 2 gene (JAK2), CALR or MPL or other genes, which make the JAK-STAT pathway overreacted. About 85% of MF patients have at least one driver gene mutation among JAK, MPL and CALR (Klampfl T, et al. NEJM 2013;369(25):2379-90;Nangalia J, et al.NEJM 2013;369(25)2391-405). In patients with primary fibromyelopathy, mutational frequency of JAK2, CALR and MPL is ~65%, ~25% and ~10% respectively. (Tefferi A. Am J Hematol. 2021 Jan;96(1):145-162.).

The treatment of Myelofibrosis (MF) is to control disease symptoms and complications, enhancing quality of life in the short term, but in the long term the treatment is to extend survival, manage/reverse Myelofibrosis (MF) even cure it. Among the current treatments for MF, traditional drugs are symptomatic treatments based on clinical manifestations, and their efficacy is limited. Currently, there are few targeted therapies for myelofibrosis. Only two targeted drugs for the treatment of myelofibrosis have been developed, which are small molecule JAK1/JAK2 kinase inhibitor ruxolitinib phosphate from Novartis and highly specific JAK2 inhibitor fedratinib from Bristol-Myers Squibb's (BMS) . At present, ruxolitinib is the only targeted drug approved in China for the treatment of myelofibrosis. But JAK inhibitors has shown some side effects in patients including thrombocytopenia, anemia, gastrointestinal disturbances, metabolic abnormalities, peripheral neuropathy, and hyperacute relapse of symptoms during treatment discontinuation.(Tefferi, A. JAK inhibitors for myeloproliferative neoplasms: clarifying facts from myths[J]. Blood, 2012, 119(12):2721-2730.).

For the above reasons, there is an urgent need to develop a drug that can effectively treat myelofibrosis and its related symptoms/signs.

SUMMARY OF THE INVENTION

The present invention is to provide a use of a compound in the preparation of a medicament for treating myelofibrosis and the symptoms/signs associated with myelofibrosis, the use of the compound as a new medicament for the treatment of myelofibrosis and the symptoms/signs associated with myelofibrosis.

In order to achieve the above purpose, according to one aspect of the present invention, there is provided use of a compound in the preparation of a medicament for treating myelofibrosis and the symptoms/signs associated with myelofibrosis, wherein the compound is selected from one or more of the compounds of formula I and the pharmaceutically acceptable salts, prodrugs, solvates and hydrates thereof:

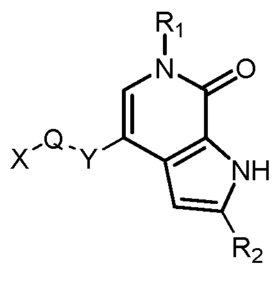

formula I

in the formula I,

$R_1$ and $R_2$ are each independently selected from H, $C_{1-6}$ alkyl, $C_{1-6}$ alkoxy, $C_{6-10}$ aryl or $C_{5-10}$ heteroaryl, wherein the $C_{1-6}$ alkyl, $C_{1-6}$ alkoxy, $C_{6-10}$ aryl or $C_{5-10}$ heteroaryl is optionally substituted with $C_{1-6}$ alkyl group, $-NH_2$ group, -OH group, $C_{6-10}$ aryl group or $C_{5-10}$ heteroaryl group; and the $C_{5-10}$ heteroaryl and the $C_{5-10}$ heteroaryl group have 1, 2, or 3 heteroatoms independently selected from nitrogen, oxygen or sulfur;

Q is absent or selected from $C_{1-6}$ alkylene, $-SO_2-$ or -NH-, wherein the $C_{1-6}$ alkylene or -NH- is optionally substituted with halogen, $C_{1-6}$ alkyl or $C_{1-6}$ alkoxy;

X is selected from H, $C_{1-6}$ alkyl, $C_{6-10}$ aryl or $C_{5-10}$ heteroaryl, wherein the $C_{1-6}$ alkyl, $C_{6-10}$ aryl or $C_{5-10}$ heteroaryl is optionally substituted by halogen, halo $C_{1-6}$ alkyl, $C_{1-6}$ alkyl, $C_{1-6}$ alkoxy, $C_{1-6}$ alkylthio, $C_{1-6}$ alkoxycarbonyl or $C_{1-6}$ alkyl-$SO_2$-;

Y is

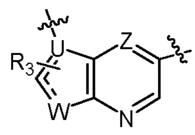
,

wherein, - - - represents a single bond or a double bond, the U, W or Z is independently selected from C or N; $R_3$ is absent or selected from H, halogen, hydroxyl, amino, $C_{1-6}$ alkyl, $C_{1-6}$ alkoxy, cyano, oxo, or $-N(R_4)-SO_2-R_5$; $R_4$ and $R_5$ are each independently selected from H, $C_{1-6}$ alkyl or halo $C_{1-6}$ alkyl.

Further more, Y is

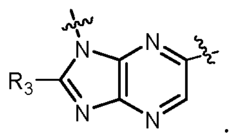
.

Further more, $R_1$ is selected from H or $C_{1-4}$ alkyl, preferably, $R_1$ is H, methyl, ethyl, propyl, isopropyl, n-butyl, sec-butyl, tert-butyl; and/or, $R_2$ is selected from H or $C_{1-3}$ alkyl; preferably, $R_2$ is H, methyl, ethyl, propyl or isopropyl; and/or, Q is absent or $C_{1-3}$ alkylene; preferably, Q is selected from methylene or ethylene; and/or, X is selected from H, $C_{1-3}$ alkyl or phenyl, alternatively, phenyl can be substituted with halogen, halo $C_{1-3}$ alkyl, $C_{1-3}$ alkyl, $C_{1-3}$ alkoxy or $C_{1-3}$ alkyl-$SO_2$-.

Further more, the compound is selected from one or more of the compound of formula II and the pharmaceutically acceptable salts, prodrugs, solvates and hydrates thereof:

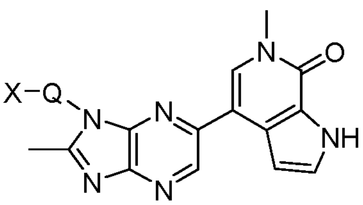

formula II

in the formula II, Q is $C_{1-6}$ alkylene; X is phenyl, the phenyl is optionally substituted with halogen, halo $C_{1-3}$ alkyl, $C_{1-3}$ alkyl, $C_{1-3}$ alkoxy or $C_{1-3}$ alkyl-$SO_2$-.

Further more, the compound is selected from one or more of:

6-methyl-4-(2-methyl-1-(4-(trifluoromethyl)benzyl)-1H-imidazo[4,5-b]pyrazin-6-yl)-1 H-pyrrolo[2,3-c]pyridin-7(6H)-one;

4-(1-(4-chlorobenzyl)-2-methyl-1H-imidazo[4,5-b]pyrazin-6-yl)-6-methyl-1H-pyrrolo[ 2,3-c]pyridin-7(6H)-one;

4-(1-(4-methoxybenzyl)-2-methyl-1H-imidazo[4,5-b]pyrazin-6-yl)-6-methyl-1H-pyrrol o[2,3-c]pyridin-7(6H)-one;

4-(1-(4-methoxybenzyl)-2-methyl-1H-imidazo[4,5-b]pyrazin-6-yl)-6-methyl-1H-pyrrol o[2,3-c]pyridin-7(6H)-one;

4-(1-benzyl-2-methyl-1H-imidazo[4,5-b]pyrazin-6-yl)-6-methyl-1H-pyrrolo[2,3-c]pyri din-7(6H)-one;

4-(1-(3-trifluoromethylbenzyl)-2-methyl-1H-imidazo[4,5-b]pyrazin-6-yl)-6-methyl-1H -pyrrolo[2,3-c]pyridin-7(6H)-one;

4-(1-(2-fluoro-5-trifluoromethylbenzyl)-2-methyl-1H-imidazo[4,5-b]pyrazin-6-yl)-6-m ethyl-1H-pyrrolo[2,3-c]pyridin-7(6H)-one;

4-(1-(3-fluoro-5-trifluoromethylbenzyl)-2-methyl-1H-imidazo[4,5-b]pyrazin-6-yl)-6-m ethyl-1H-pyrrolo[2,3-c]pyridin-7(6H)-one;

4-(1-(2-fluoro-4-chlorobenzyl)-2-methyl-1H-imidazo[4,5-b]pyrazin-6-yl)-6-methyl-1H -pyrrolo[2,3-c]pyridin-7(6H)-one;

4-(1-(3-trifluoromethyl-4-chlorobenzyl)-2-methyl-1H-imidazo[4,5-b]pyrazin-6-yl)-6-m ethyl-1H-pyrrolo[2,3-c]pyridin-7(6H)-one;

4-(1-(3-fluoro-4-trifluoromethylbenzyl)-2-methyl-1H-imidazo[4,5-b]pyrazin-6-yl)-6-m ethyl-1H-pyrrolo[2,3-c]pyridin-7(6H)-one;

4-(1-(3-chloro-4-trifluoromethylbenzyl)-2-methyl-1H-imidazo[4,5-b]pyrazin-6-yl)-6-m ethyl-1H-pyrrolo[2,3-c]pyridin-7(6H)-one;

4-(1-(3-chlorobenzyl)-2-methyl-1H-imidazo[4,5-b]pyrazin-6-yl)-6-methyl-1H-pyrrolo[ 2,3-c]pyridin-7(6H)-one;

4-(1-(2,4-difluorobenzyl)-2-methyl-1H-imidazo[4,5-b]pyrazin-6-yl)-6-methyl-1H-pyrr olo[2,3-c]pyridin-7(6H)-one;

4-(1-(4-bromobenzyl)-2-methyl-1H-imidazo[4,5-b]pyrazin-6-yl)-6-methyl-1H-pyrrolo[ 2,3-c]pyridin-7(6H)-one;

6-methyl-4-(2-methyl-1-(4-(methylsulfonyl)benzyl)-1H-imidazo[4,5-b]pyrazin-6-yl)-1 H-pyrrolo[2,3-c]pyridin-7(6H)-one;

preferably, the compound is 6-methyl-4-(2-methyl-1-(4-(trifluoromethyl)benzyl)-1H-imidazo[4,5-b]pyrazin-6-yl)-1H-py rrolo[2,3-c]pyridin-7(6H)-one.

Further more, the medicament also comprising excipients.

Further more, the medicament is administered intravenously, intramuscularly, parenterally, nasally, orally or rectally.

According to another aspect of the present invention, there is also provided a method of treating myelofibrosis and the symptoms/signs associated with myelofibrosis, wherein the method comprising: providing a medicament comprising the compound, wherein the compound is selected from one or more of the compound of formula I and the pharmaceutically acceptable salts, prodrugs, solvates and hydrates thereof; and administering therapeutically effective amount of the medicament to subject having myelofibrosis;

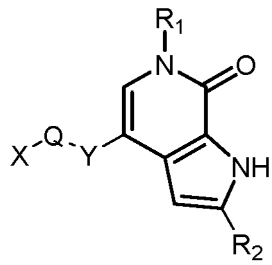

formula I

in the formula I,

$R_1$ and $R_2$ are each independently selected from H, $C_{1-6}$ alkyl, $C_{1-6}$ alkoxy, $C_{6-10}$ aryl or $C_{5-10}$ heteroaryl, wherein the $C_{1-6}$ alkyl, $C_{1-6}$ alkoxy, $C_{6-10}$ aryl or $C_{5-10}$ heteroaryl is optionally substituted with $C_{1-6}$ alkyl group, $-NH_2$ group, -OH group, $C_{6-10}$ aryl group or $C_{5-10}$ heteroaryl group; and the $C_{5-10}$ heteroaryl and the $C_{5-10}$ heteroaryl group has 1, 2, or 3 heteroatoms independently selected from nitrogen, oxygen or sulfur;

Q is absent or selected from $C_{1-6}$ alkylene, $-SO_2-$ or -NH-, wherein the $C_{1-6}$ alkylene or -NH- is optionally substituted with halogen, $C_{1-6}$ alkyl or $C_{1-6}$ alkoxy;

X is selected from H, $C_{1-6}$ alkyl, $C_{6-10}$ aryl or $C_{5-10}$ heteroaryl, wherein the $C_{1-6}$ alkyl, $C_{6-10}$ aryl or $C_{5-10}$ heteroaryl is optionally substituted by halogen, halo $C_{1-6}$ alkyl, $C_{1-6}$ alkyl, $C_{1-6}$ alkoxy, $C_{1-6}$ alkylthio, $C_{1-6}$ alkoxycarbonyl or $C_{1-6}$ alkyl-$SO_2$-;

Y is

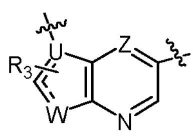

,

wherein, - - - represents a single bond or a double bond, the U, W or Z is independently selected from C or N; $R_3$ is absent or selected from H, halogen, hydroxyl, amino, $C_{1-6}$ alkyl, $C_{1-6}$ alkoxy, cyano, oxo, or $-N(R_4)-SO_2-R_5$; $R_4$ and $R_5$ are each independently selected from H, $C_{1-6}$ alkyl or halo $C_{1-6}$ alkyl.

Further more, Y is

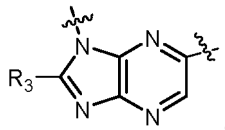

.

Further more, $R_1$ is selected from H or $C_{1-4}$ alkyl, preferably, $R_1$ is H, methyl, ethyl, propyl, isopropyl, n-butyl, sec-butyl, tert-butyl; and/or, $R_2$ is selected from H or $C_{1-3}$ alkyl; preferably, $R_2$ is H, methyl, ethyl, propyl or isopropyl; and/or, Q is absent or $C_{1-3}$ alkylene; preferably, Q is selected from methylene or ethylene; and/or, X is selected from H, $C_{1-3}$ alkyl or phenyl, alternatively, phenyl can be substituted with halogen, halo $C_{1-3}$ alkyl, $C_{1-3}$ alkyl, $C_{1-3}$ alkoxy or $C_{1-3}$ alkyl-$SO_2$-.

Further more, the compound is selected from one or more of the compound of formula II and the pharmaceutically acceptable salts, prodrugs, solvates and hydrates thereof:

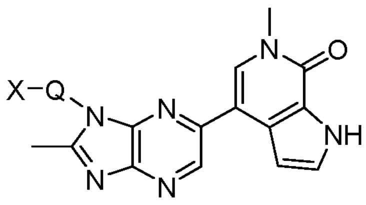

formula II

in the formula II, Q is $C_{1-6}$ alkylene; X is phenyl, the phenyl is optionally substituted with halogen, halo $C_{1-3}$ alkyl, $C_{1-3}$ alkyl, $C_{1-3}$ alkoxy or $C_{1-3}$ alkyl-$SO_2$-.

Further more, the compound is selected from one or more of:

6-methyl-4-(2-methyl-1-(4-(trifluoromethyl)benzyl)-1H-imidazo[4,5-b]pyrazin-6-yl)-1 H-pyrrolo[2,3-c]pyridin-7(6H)-one;

4-(1-(4-chlorobenzyl)-2-methyl-1H-imidazo[4,5-b]pyrazin-6-yl)-6-methyl-1H-pyrrolo[ 2,3-c]pyridin-7(6H)-one;

4-(1-(4-methoxybenzyl)-2-methyl-1H-imidazo[4,5-b]pyrazin-6-yl)-6-methyl-1H-pyrrol o[2,3-c]pyridin-7(6H)-one;

4-(1-(4-methoxybenzyl)-2-methyl-1H-imidazo[4,5-b]pyrazin-6-yl)-6-methyl-1H-pyrrol o[2,3-c]pyridin-7(6H)-one;

4-(1-benzyl-2-methyl-1H-imidazo[4,5-b]pyrazin-6-yl)-6-methyl-1H-pyrrolo[2,3-c]pyri din-7(6H)-one;

4-(1-(3-trifluoromethylbenzyl)-2-methyl-1H-imidazo[4,5-b]pyrazin-6-yl)-6-methyl-1H -pyrrolo[2,3-c]pyridin-7(6H)-one;

4-(1-(2-fluoro-5-trifluoromethylbenzyl)-2-methyl-1H-imidazo[4,5-b]pyrazin-6-yl)-6-m ethyl-1H-pyrrolo[2,3-c]pyridin-7(6H)-one;

4-(1-(3-fluoro-5-trifluoromethylbenzyl)-2-methyl-1H-imidazo[4,5-b]pyrazin-6-yl)-6-m ethyl-1H-pyrrolo[2,3-c]pyridin-7(6H)-one;

4-(1-(2-fluoro-4-chlorobenzyl)-2-methyl-1H-imidazo[4,5-b]pyrazin-6-yl)-6-methyl-1H -pyrrolo[2,3-c]pyridin-7(6H)-one;

4-(1-(3-trifluoromethyl-4-chlorobenzyl)-2-methyl-1H-imidazo[4,5-b]pyrazin-6-yl)-6-m ethyl-1H-pyrrolo[2,3-c]pyridin-7(6H)-one;

4-(1-(3-fluoro-4-trifluoromethylbenzyl)-2-methyl-1H-imidazo[4,5-b]pyrazin-6-yl)-6-m ethyl-1H-pyrrolo[2,3-c]pyridin-7(6H)-one;

4-(1-(3-chloro-4-trifluoromethylbenzyl)-2-methyl-1H-imidazo[4,5-b]pyrazin-6-yl)-6-m ethyl-1H-pyrrolo[2,3-c]pyridin-7(6H)-one;

4-(1-(3-chlorobenzyl)-2-methyl-1H-imidazo[4,5-b]pyrazin-6-yl)-6-methyl-1H-pyrrolo[ 2,3-c]pyridin-7(6H)-one;

4-(1-(2,4-difluorobenzyl)-2-methyl-1H-imidazo[4,5-b]pyrazin-6-yl)-6-methyl-1H-pyrr olo[2,3-c]pyridin-7(6H)-one;

4-(1-(4-bromobenzyl)-2-methyl-1H-imidazo[4,5-b]pyrazin-6-yl)-6-methyl-1H-pyrrolo[ 2,3-c]pyridin-7(6H)-one;

6-methyl-4-(2-methyl-1-(4-(methylsulfonyl)benzyl)-1H-imidazo[4,5-b]pyrazin-6-yl)-1 H-pyrrolo[2,3-c]pyridin-7(6H)-one;

preferably, the compound is 6-methyl-4-(2-methyl-1-(4-(trifluoromethyl)benzyl)-1H-imidazo[4,5-b]pyrazin-6-yl)-1H-py rrolo[2,3-c]pyridin-7(6H)-one.

Further more, the myelofibrosis is selected from the group consisting of primary myelofibrosis, Post-Polycythemia Vera Myelofibrosis or Post-Essential Thrombocythemia Myelofibrosis.
Further more, the medicament also comprising excipients.
Further more, the medicament is administered intravenously, intramuscularly, parenterally, nasally, orally or rectally.
Further more, the compound is administered in a dosage of 1~500mg/day.
Further more, the compound is administered orally in a dosage of about 3mg/day, or 5mg/ day, or 10mg/ day, or 20mg/ day, or 25mg/ day, or 30mg/ day, or 35mg/ day, or 40mg/ day, or 45mg/ day, or 50mg/ day, or 55mg/ day, or 60mg/ day, or 70mg/ day, or 80mg/ day, or 90mg/ day, or 100mg/ day, or 150mg/ day, or 200mg/ day.
The present invention provides a use of the compound in the preparation of a medicament for treating myelofibrosis and the symptoms/signs associated with myelofibrosis; the compound is selected from one or more of the compound of formula I and the pharmaceutically acceptable salts, prodrugs, solvates and hydrates thereof:

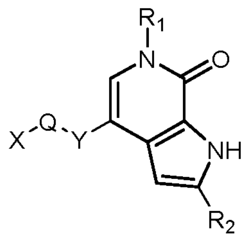

formula I

the compound has a significant inhibitory effect on the proliferation of human myeloproliferative tumor cells HEL and SET-2 cells with JAK2V617F mutation phenotype, and also has a good inhibitory effect on the transcriptional activity of NF-κB and the secretion of the inflammatory factor IL-6, and can be useful in the treatment of myelofibrotic disease and the symptoms/signs associated with myelofibrosis.

## DESCRIPTION OF THE DRAWINGS

The drawings that constitute a part of the present invention are used to provide a further understanding, the illustrative examples and there descriptions are used to explain the present invention and do not constitute an improper limitation of the present invention. In the drawings:

Figure 1 shows the proliferation inhibition curve of compound 1 against HEL cells at different concentrations in Example 2;

Figure 2 shows the proliferation inhibition curve of compound 1 against SET-2 cells at different concentrations in Example 2;

Figure 3 shows the inhibition curve of compound 1 on NF-κB transcriptional activity at different concentrations in Example 3;

Figure 4 shows the inhibitory curve of compound 1 on IL-6 in THP-1 cells at different concentrations in Example 4.

## EXAMPLES

It should be noted that, as long as there is no conflict, the examples and features in the examples of the present invention can be combined with each other. The present invention will be described in detail below with reference to the drawings and examples.

Definitions:

In the present invention, unless otherwise stated, the term "halogen" refers to fluorine, chlorine, bromine or iodine. Preferably, halogen refers to fluorine, chlorine and bromine.
In the present invention, unless otherwise stated, the term "alkyl" includes a straight, branched or cyclic saturated monovalent hydrocarbon. For example, alkyl includes methyl, ethyl, propyl, isopropyl, cyclopropyl, n-butyl, isobutyl, sec-butyl, tert-butyl, cyclobutyl, n-pentyl, 3-(2-methyl)butyl, 2-pentyl, 2-methylbutyl, neopentyl, cyclopentyl, n-hexyl, 2-hexyl, 2-methylpentyl and cyclohexyl. Similarly, "$C_{1-6}$" in $C_{1-6}$ alkyl refers to a group containing 1, 2, 3, 4, 5 or 6 carbon atoms arranged in a linear or branched chain.

In the present invention, unless otherwise stated, the term "heteroaryl" refers to a substituted or unsubstituted stable monocyclic or bicyclic group containing at least one aromatic ring of 5 to 10 ring atoms, the aromatic ring containing one, two or three ring heteroatoms selected from N, O and S, the remaining ring atoms are C atoms. Examples of such heteroaryl groups include, but are not limited to, pyridyl, pyrimidinyl, pyrrolyl, imidazolyl, thiazolyl, thienyl, and benzimidazole.

In the present invention, the term "prodrugs" refers to functional derivatives of the compounds that are readily converted in vivo into the required compound. Thus, in the methods for the treatment of the present invention, the term "administering" shall encompass the treatment of the various disorders described with the compound specifically disclosed or with a compound which may not be specifically disclosed, but which converts to the specified compound in vivo after administration to the subject, for the treatment of myelofibrosis and the symptoms/signs associated with myelofibrosis.

When the compound of Formula I and pharmaceutically acceptable salts thereof exist in the form of solvates or polymorphic forms, the "compound" of the present invention includes any possible solvates and polymorphic forms. A type of a solvent that forms the solvate is not particularly limited so long as the solvent is pharmacologically acceptable. For example, water, ethanol, propanol, acetone and similar solvents can be used.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, manganese (ic and ous), potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include ion exchange resins such as, for example, arginine, betaine, caffeine, choline, N',N'- dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like. When the compound of the present invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Preferred are citric, hydrobromic, formic, hydrochloric, maleic, phosphoric, sulfuric and tartaric acids, particularly preferred are formic and hydrochloric acid. Since the compounds of Formula I are intended for pharmaceutical use they are preferably provided in substantially pure form, for example at least 60% pure, more suitably at least 75% pure, especially at least 98% pure (% are on a weight for weight basis).

In order to treat myelofibrosis and its associated symptoms/signs with medicament, the present invention provides a use of the compound in the preparation of a medicament for treating myelofibrosis and the symptoms/signs associated with myelofibrosis, and provides a method of treating myelofibrosis and the symptoms/signs associated with myelofibrosis. The above method comprising: providing a medicament comprising the compound, and administering therapeutically effective amount of the medicament to subject having myelofibrosis. The above compound is selected from one or more of the compound of formula I and the pharmaceutically acceptable salts, prodrugs, solvates and hydrates thereof:

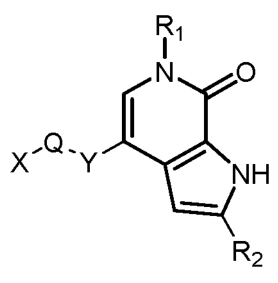

formula I

in the formula I,

$R_1$ and $R_2$ are each independently selected from H, $C_{1-6}$ alkyl, $C_{1-6}$ alkoxy, $C_{6-10}$ aryl or $C_{5-10}$ heteroaryl, wherein the $C_{1-6}$ alkyl, $C_{1-6}$ alkoxy, $C_{6-10}$ aryl or $C_{5-10}$ heteroaryl is optionally substituted with $C_{1-6}$ alkyl group, $-NH_2$ group, -OH group, $C_{6-10}$ aryl group or $C_{5-10}$ heteroaryl group; and the $C_{5-10}$ heteroaryl and the $C_{5-10}$ heteroaryl group have 1, 2, or 3 heteroatoms independently selected from nitrogen, oxygen or sulfur;
Q is absent or selected from $C_{1-6}$ alkylene, $-SO_2-$ or -NH-, wherein the $C_{1-6}$ alkylene or -NH- is optionally substituted with halogen, $C_{1-6}$ alkyl or $C_{1-6}$ alkoxy;
X is selected from H, $C_{1-6}$ alkyl, $C_{6-10}$ aryl or $C_{5-10}$ heteroaryl, wherein the $C_{1-6}$ alkyl, $C_{6-10}$ aryl or $C_{5-10}$ heteroaryl is optionally substituted by halogen, halo $C_{1-6}$ alkyl, $C_{1-6}$ alkyl, $C_{1-6}$ alkoxy, $C_{1-6}$ alkylthio, $C_{1-6}$ alkoxycarbonyl or $C_{1-6}$ alkyl-$SO_2$-;
Y is

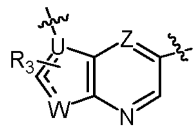,

wherein, - - - represents a single bond or a double bond, the U, W or Z is independently selected from C or N; $R_3$ is absent or selected from H, halogen, hydroxyl, amino, $C_{1-6}$ alkyl, $C_{1-6}$ alkoxy, cyano, oxo, or $-N(R_4)-SO_2-R_5$; $R_4$ and $R_5$ are each independently selected from H, $C_{1-6}$ alkyl or halo $C_{1-6}$ alkyl.

The inventors of the present invention unexpectedly discovered that the compound of Formula I has a significant inhibitory effect on the proliferation of human myeloproliferative tumor cells HEL and SET-2 cells with JAK2V617F mutation phenotype, and also has a good inhibitory effect on the transcriptional activity of NF-κB and the secretion of the inflammatory factor IL-6, its associated symptoms/signs, and can be useful in the treatment of myelofibrotic disease and its associated symptoms/signs.

In addition, it should be noted that, the Y group of the aboved compound of formula I is required to be

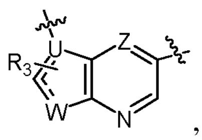,

compared to other 5- and/or 6-membered merged rings, such as

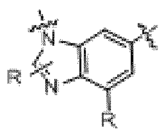,

the compound of formula I of the present invention has significant differences and obvious effectiveness in the treatment of myelofibrosis. More preferably, Y is

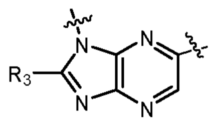.

In order to further improving the effectiveness of treatment of myelofibrosis and the symptoms/signs associated with myelofibrosis, in the preferred embodiment, $R_1$ is selected from H or $C_{1-4}$ alkyl, preferably, $R_1$ is H, methyl, ethyl, propyl, isopropyl, n-butyl, sec-butyl, tert-butyl; more preferably, $R_1$ is methyl.

In a preferred embodiment, $R_2$ is selected from H or $C_{1-3}$ alkyl; preferably, $R_2$ is H, methyl, ethyl, propyl or isopropyl; more preferably, $R_2$ is H.

In a preferred embodiment, Q is absent or $C_{1-3}$ alkylene; more preferably, Q is selected from methylene or

ethylene; more preferably, Q is methylene.

In a preferred embodiment, X is selected from H, $C_{1-3}$ alkyl or phenyl, alternatively, phenyl can be substituted with halogen, halo $C_{1-3}$ alkyl, $C_{1-3}$ alkyl, $C_{1-3}$ alkoxy or $C_{1-3}$ alkyl-$SO_2$-.

In a preferred embodiment, the compound is selected from one or more of the compound of formula II and the pharmaceutically acceptable salts, prodrugs, solvates and hydrates thereof:

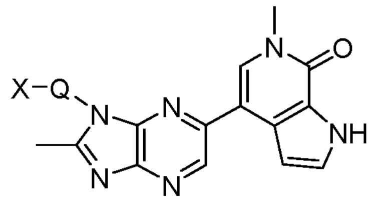

formula II

in the formula II, Q is $C_{1-6}$ alkylene; X is phenyl, the phenyl is optionally substituted with halogen, halo $C_{1-3}$ alkyl, $C_{1-3}$ alkyl, $C_{1-3}$ alkoxy or $C_{1-3}$ alkyl-$SO_2$-.

Illustratively, the above compounds are selected from one or more of:

6-methyl-4-(2-methyl-1-(4-(trifluoromethyl)benzyl)-1H-imidazo[4,5-b]pyrazin-6-yl)-1 H-pyrrolo[2,3-c]pyridin-7(6H)-one;

4-(1-(4-chlorobenzyl)-2-methyl-1H-imidazo[4,5-b]pyrazin-6-yl)-6-methyl-1H-pyrrolo[ 2,3-c]pyridin-7(6H)-one;

4-(1-(4-methoxybenzyl)-2-methyl-1H-imidazo[4,5-b]pyrazin-6-yl)-6-methyl-1H-pyrrol o[2,3-c]pyridin-7(6H)-one;

4-(1-(4-methoxybenzyl)-2-methyl-1H-imidazo[4,5-b]pyrazin-6-yl)-6-methyl-1H-pyrrol o[2,3-c]pyridin-7(6H)-one;

4-(1-benzyl-2-methyl-1H-imidazo[4,5-b]pyrazin-6-yl)-6-methyl-1H-pyrrolo[2,3-c]pyri din-7(6H)-one;

4-(1-(3-trifluoromethylbenzyl)-2-methyl-1H-imidazo[4,5-b]pyrazin-6-yl)-6-methyl-1H -pyrrolo[2,3-c]pyridin-7(6H)-one;

4-(1-(2-fluoro-5-trifluoromethylbenzyl)-2-methyl-1H-imidazo[4,5-b]pyrazin-6-yl)-6-m ethyl-1H-pyrrolo[2,3-c]pyridin-7(6H)-one;

4-(1-(3-fluoro-5-trifluoromethylbenzyl)-2-methyl-1H-imidazo[4,5-b]pyrazin-6-yl)-6-m ethyl-1H-pyrrolo[2,3-c]pyridin-7(6H)-one;

4-(1-(2-fluoro-4-chlorobenzyl)-2-methyl-1H-imidazo[4,5-b]pyrazin-6-yl)-6-methyl-1H -pyrrolo[2,3-c]pyridin-7(6H)-one;

4-(1-(3-trifluoromethyl-4-chlorobenzyl)-2-methyl-1H-imidazo[4,5-b]pyrazin-6-yl)-6-m ethyl-1H-pyrrolo[2,3-c]pyridin-7(6H)-one;

4-(1-(3-fluoro-4-trifluoromethylbenzyl)-2-methyl-1H-imidazo[4,5-b]pyrazin-6-yl)-6-m ethyl-1H-pyrrolo[2,3-c]pyridin-7(6H)-one;

4-(1-(3-chloro-4-trifluoromethylbenzyl)-2-methyl-1H-imidazo[4,5-b]pyrazin-6-yl)-6-m ethyl-1H-pyrrolo[2,3-c]pyridin-7(6H)-one;

4-(1-(3-chlorobenzyl)-2-methyl-1H-imidazo[4,5-b]pyrazin-6-yl)-6-methyl-1H-pyrrolo[ 2,3-c]pyridin-7(6H)-one;

4-(1-(2,4-difluorobenzyl)-2-methyl-1H-imidazo[4,5-b]pyrazin-6-yl)-6-methyl-1H-pyrr olo[2,3-c]pyridin-7(6H)-one;

4-(1-(4-bromobenzyl)-2-methyl-1H-imidazo[4,5-b]pyrazin-6-yl)-6-methyl-1H-pyrrolo[ 2,3-c]pyridin-7(6H)-one;

6-methyl-4-(2-methyl-1-(4-(methylsulfonyl)benzyl)-1H-imidazo[4,5-b]pyrazin-6-yl)-1 H-pyrrolo[2,3-c]pyridin-7(6H)-one;

preferably, the compound is 6-methyl-4-(2-methyl-1-(4-(trifluoromethyl)benzyl)-1H-imidazo[4,5-b]pyrazin-6-yl)-1H-py rrolo[2,3-c]pyridin-7(6H)-one.

In one embodiment of the invention, the symptoms/signs associated with myelofibrosis is one or more of the following: anemia, splenomegaly, hepatomegaly, fatigue, early satiety, abdominal discomfort, inactivity, inability to concentrate, night sweats, itching, diffuse bone pain, fever, and weight loss.

In practical applications, the medicament also comprises excipients. Specific excipients include, but not limited to, pharmaceutically acceptable carriers, excipients or adjuvants, etc. Moreover, the medicament can be prepared into different dosage forms to adapt to diverse administration route, the specific suitable administration routes include intravenous, intramuscular, parenterally, nasally, orally or rectally. The dosage forms of the medicament include tablets, capsules, pills, powders, suppositories, solutions, suspensions, aerosols, emulsions, ointments, lotions, dusting powders or other similar dosage forms.

The pharmaceutical carrier employed can be, for example, a solid, liquid, or gas. Examples of solid carriers include such as lactose, terra alba, sucrose, talc, gelatin, agar, pectin, acacia, magnesium stearate, and stearic acid. Examples of liquid carriers include such as sugar syrup, peanut oil, olive oil, and water. Examples of gaseous carriers include such as carbon dioxide and nitrogen. In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

The above tablet may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.05mg to about 5g of the active ingredient and each cachet or capsule preferably containing from about 0.05mg to about 5g of the active ingredient. For example, a formulation intended for the oral administration to humans may contain from about 0.5mg to about 5g of active agent, compounded with an appropriate and convenient amount of carrier material which may vary from about 5 to about 95 percent of the total composition. Unit dosage forms will generally contain between from about 1 mg to about 2g of the active ingredient, typically 1mg, 1.5mg, 2mg, 2.5mg, 3mg, 5mg, 15mg, 20mg, 25mg, 50mg, 100mg, 200mg, 300mg, 400mg, 500mg, 600mg, 800mg or 1000mg.

The above mentioned medicament suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

The above mentioned medicament suitable for injectable use includes sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), vegetable oils, and suitable mixtures thereof.

The above mentioned medicament can be in a form suitable for rectal administration with solid as a carrier. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including antioxidants) and the like. Furthermore, other adjuvants can be included to render the formulation isotonic with the blood of the intended recipient. Compositions containing a compound described by Formula I, or pharmaceutically acceptable salts thereof, may also be prepared in powder or liquid concentrate form.

The term "effective amount" is therapeutically effective amount. The "therapeutically effective amount" is an

amount sufficient to provide a desired therapeutic result over the necessary dosage and specified duration. The therapeutically effective amount of the medicament can vary depending on various factors, for example, the individual's condition, age, gender, and weight, as well as the ability of drugs to elicit the desired response in the individual's body. The therapeutically effective amount is also the amount in which the therapeutically beneficial effects outweigh any toxic or detrimental effects of the subject. In one embodiment, in the method of treating myelofibrosis and the symptoms/signs associated with myelofibrosis provided by the present invention, the above compound is administered in dosage of 1~500mg/day, preferably the dosage of the compound is 3 ~200mg/day, preferably the dosage of the compound is 5~150mg/day. Exemplarily, the above compound is administered in dosage of about 3 mg/day, or 5 mg/day, or 10 mg/day, or 20 mg/day, or 25 mg/day, or 30 mg/day, or 35 mg/day, or 40 mg/day, or 45 mg/day, or 50 mg/day, or 55 mg/day, or 60 mg/day, or 70 mg/day, or 80 mg/day, or 90 mg/day, or 100 mg/day, or 150 mg/day, or 200 mg/dayfor oral administration.

The present invention will be described in detail below in conjunction with examples, which are merely illustrative and not limited to the scope of application of the present invention.

Abbreviations:

ALT: Alanine Aminotransferase;

ANC: Absolute Neutrophil Count;

APTT: Activated Partial Thromboplastin Time;

AST: Aspartate Transaminase;

BID: Twice A Day;

CI: Clinical Improvement;

CT: Computed Tomography;

CR: Complete Response;

Ccr: Creatinine Clearance;

cm: Centimeter;

DIEA: N,N-diisopropylethylamine;

DIPSS: Dynamic International Prognostic Scoring System;

DLTs/DLT: Dose-Limiting Toxicities;

DMSO: Dimethyl Sulfoxide;

DCM: Dichloromethane;

EA: Ethyl Acetate;

ECOG: Eastern Cooperative Oncology Group;

ELN: European Leukemia Network;

HGB: Hemoglobin;

INR: International Normalized Ratio;

IWG-MRT: International Working Group-Myeloproliferative Neoplasms Research and Treatment;

LCMS/LC-MS: Liquid Chromatography Coupled With Mass Spectrometry;

MF: Myelofibrosis;

MPN: Myeloproliferative Neoplasm;

MPN-SAF: MPN Symptom Assessment Form;

MPN-SAF TSS: MPN Symptom Assessment Form-Total Symptom Score;

MTD: Maximum Tolerated Dose;

MRI: Nuclear Magnetic Resonance;

mg: Milligram;

mL: Milliliter;

mM: Millimole Per Liter;

nM: Nanomoles Per Liter;

μM: Micromoles Per Liter;

NCI CTCAE: National Cancer Institute Common Terminology Criteria for Adverse Events;

$Pd(dppf)Cl_2$.DCM: 1,1'-bis(diphenylphosphine)ferrocene]Palladium Dichloride Dichloromethane Complex;

RP2D: Phase II recommended Dose;

PE: Petroleum Ether;

PMF: Primary Myelofibrosis;

post-PV MF: Post-Polycythemia Vera Myelofibrosis;

post-ET MF: Post-Essential Thrombocythemia Myelofibrosis;

PLT: Platelet;

PR: Partial Response;

PD: Progressive Disease;

Scr: Serum Creatinine;

SD: Stable Disease;

TBIL: Serum Total Bilirubin;

ULN: Upper Limit Of Normal;

WHO: World Health Organization.

**Example 1**

Preparation of 6-methyl-4-(2-methyl-1-(4-(trifluoromethyl)benzyl)-1H-imidazo[4,5-b]pyrazin-6-yl)-1H-py rrolo[2,3-c]pyridin-7(6H)-one

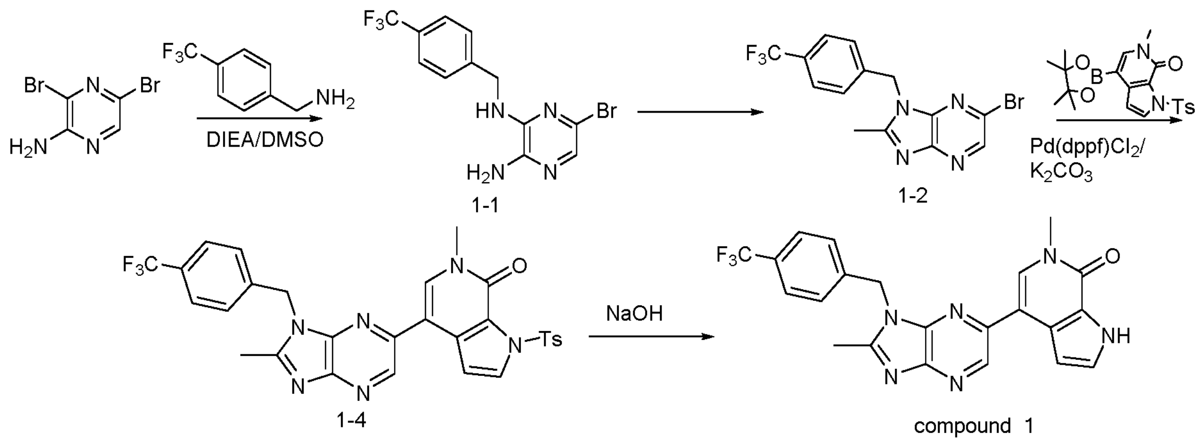


1-1 1-2 1-4 compound 1

Step1, synthesis of compound 1-1

2-amino-3,5-dibromopyrazine (10.00g), 4-trifluoromethylbenzylamine (20.98g) and DIEA (25.54g) was dissolved in DMSO (60mL), heated and stirred at 120 °C for 4h, LCMS confirmed the end of the reaction. Cooled, cold water (250mL) was added, extracted 3 times with EA, combined organic phases, washed with saturated brine 3 times, dried with anhydrous sodium sulfate, concentrated, and purified the crude product by column chromatography(the mobile phase is PE:EA=100:15-100:30) to obtain compound 1-1(15.01g).

LCMS: $[M+1]^+$ =347.0

Step2, synthesis of compound 1-2

Compound 1-1 (15.43g), triethyl orthoacetate (35.96g) and glacial acetic acid (200mL) were mixed and reacted at 100 °C overnight. Cooled, concentrated, diluted the crude product with EA, wash 3 times with saturated $Na_2CO_3$ solution, 3 times with saturated brine, dried over anhydrous sodium sulfate, and concentrated. The crude product was purified by column chromatography (the mobile phase is PE:EA=100:20-100:50) to obtain compound 1-2 (13.27g).

LCMS: $[M+1]^+$ =371.0

Step3, synthesis of compound 1-4

Compound 1-2 (1.10g), 2M-1(1.27g), $Pd(dppf)Cl_2$.DCM(0.25g) were dissolved in dioxane (20mL), $K_2CO_3$ (0.61g) and water (4mL) were added, protected by nitrogen, heated and stirred at 100 °C overnight. Cooled, the reaction solution was poured into a mixed solvent of EA (50 mL) and saturated $Na_2CO_3$ (50 mL), separated the liquid, aqueous phase was extracted 3 times with EA, organic phases were combined, washed with saturated brine 3 times, dried over anhydrous sodium sulfate, and concentrated, the crude product was purified by column chromatography (the mobile phase is DCM:MeOH=100:2) to obtain compound 1-4 (1g ).

LCMS: $[M+1]^+$ =593.1

Step4, synthesis of compound 1

Compound 1-4 (20.00g) was dissolved in EtOH (80mL) and DCM (250mL), NaOH (2.70g) was added, stirred at 60 °C overnight, concentrated. The crude product was purified by column chromatography (the mobile phase is DCM:MeOH=100:2) to obtain a brownish-yellow crude product, the brownish-yellow crude product was slurried with DCM (40mL), the solid was collected by filtration, and dried under reduced pressure to give compound 1 as a off-white solid (6.53g).

LCMS: $[M+1]^+$ =439.1.

$^1$H NMR (400 MHz, DMSO) δ 12.13 (s, 1H), 8.92 (s, 1H), 7.99 (d, J = 35.1 Hz, 1H), 7.76 (t, J = 17.0 Hz, 2H), 7.49 (d, J = 8.0 Hz, 2H), 7.28 (s, 1H), 6.75 (s, 1H), 5.68 (s, 2H), 3.63 (s, 3H), 2.63 (s, 3H).

Using a method basically similar to that of Example 1, the corresponding trifluoromethylbenzylamine derivative is used to replace

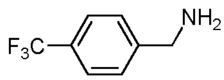

(trifluoromethylbenzylamine) to prepare the example in Table 1 below. The corresponding trifluoromethylbenzylamine derivative, such as

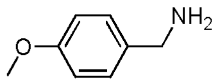 , 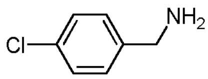 ,

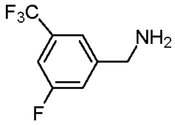 , 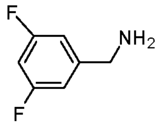 or 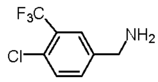

etc, can all be purchased through commercially available channels.

Table 1

| Ex. No. | Structure | Chemical Name | Physical data (MS) (M+H)$^+$ |
|---|---|---|---|
| 2 | 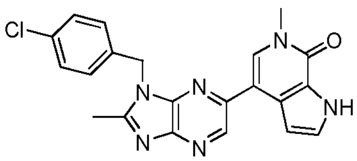 | 4-(1-(4-chlorobenzyl)-2-meth yl-1H-imidazo[4,5-b]pyrazin-6-yl)-6-methyl-1H-pyrrolo[2, 3-c] pyridin-7(6H)-one | 405.8 |
| 3 | 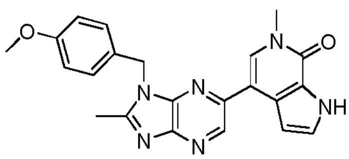 | 4-(1-(4-methoxybenzyl)-2-me thyl-1H-imidazo [4,5-b]pyrazi n-6-yl)-6-methyl-1H-pyrrolo[ 2,3-c] pyridin-7(6H)-one | 401.2 |
| 4 | 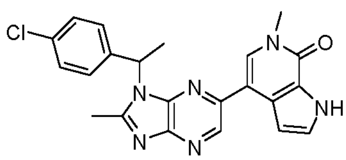 | 4-(1-(1-(4-chlorophenyl)ethyl )-2-methyl-1H-imidazo[4,5-b ]pyrazin-6-yl)-6-methyl-1,6-d ihydro-7H-pyrrolo[2,3-c]pyri din-7-one | 419.1 |
| 5 | 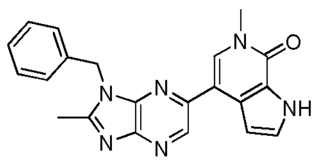 | 4-(1-benzyl-2-methyl-1H-imi dazo[4,5-b]pyrazin-6-yl)-6-m ethyl-1H-pyrrolo[2,3-c]pyridi n-7 (6H)-one | 371.1 |

(continued)

| Ex. No. | Structure | Chemical Name | Physical data (MS) $(M+H)^+$ |
|---|---|---|---|
| 6 | 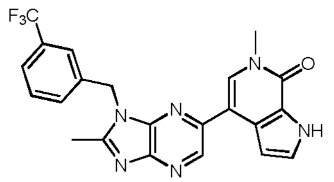 | 4-(1-(3-trifluoromethylbenzyl )-2-methyl-1H-imidazo[4,5-b ]pyrazin-6-yl)-6-methyl-1H-p yrrolo[2,3-c]pyridin-7(6H)-on e | 439.1 |
| 7 | 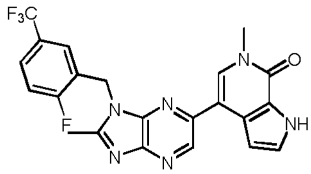 | 4-(1-(2-fluoro-5-trifluoromet hylbenzyl)-2-methyl-1H-imid azo[4,5-b]pyrazin-6-yl)-6-met hyl-1H-pyrrolo[2,3-c]pyridin-7(6H)-one | 457.1 |
| 8 | 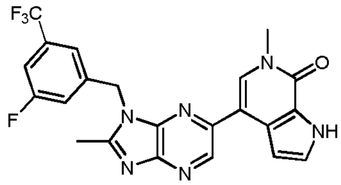 | 4-(1-(3-fluoro-5-trifluoromet hylbenzyl)-2-methyl-1H-imid azo[4,5-b]pyrazin-6-yl)-6-met hyl-1H-pyrrolo[2,3-c]pyridin-7(6H)-one | 457.1 |
| 9 | 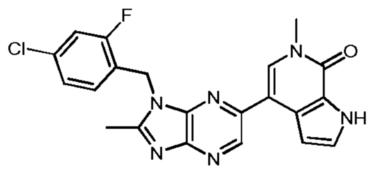 | 4-(1-(2-fluoro-4-chlorobenzyl )-2-methyl-1H-imidazo[4,5-b ]pyrazin-6-yl)-6-methyl-1H-p yrrolo[2,3-c]pyridin-7(6H)-on e | 423.1 |
| 10 | 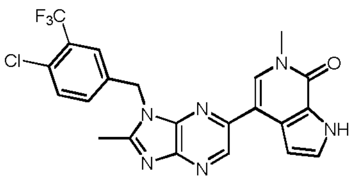 | 4-(1-(3-trifluoromethyl-4-chl orobenzyl)-2-methyl-1H-imid azo[4,5-b]pyrazin-6-yl)-6-met hyl-1H-pyrrolo[2,3-c]pyridin-7(6H)-one | 473.1 |
| 11 | 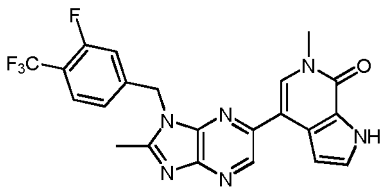 | 4-(1-(3-fluoro-4-trifluoromet hylbenzyl)-2-methyl-1H-imid azo[4,5-b]pyrazin-6-yl)-6-met hyl-1H-pyrrolo[2,3-c]pyridin-7(6H)-one | 457.1 |
| 12 | 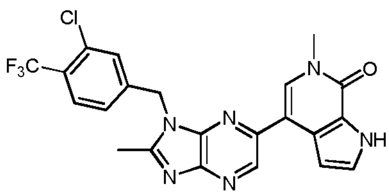 | 4-(1-(3-chloro-4-trifluoromet hylbenzyl)-2-methyl-1H-imid azo[4,5-b]pyrazin-6-yl)-6-met hyl-1H-pyrrolo[2,3-c]pyridin-7(6H)-one | 473.1 |
| 13 | 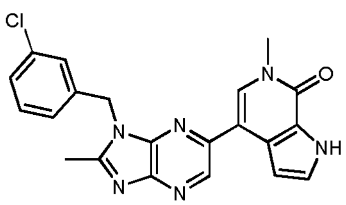 | 4-(1-(3-chlorobenzyl)-2-meth yl-1H-imidazo[4,5-b]pyrazin-6-yl)-6-methyl-1H-pyrrolo[2, 3-c] pyridin-7(6H)-one | 405.1 |

(continued)

| Ex. No. | Structure | Chemical Name | Physical data (MS) (M+H)+ |
|---|---|---|---|
| 14 | 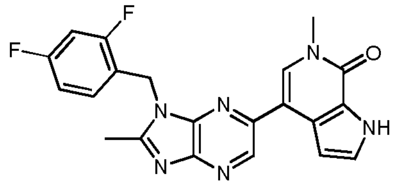 | -(1-(2,4-difluorobenzyl)-2-me thyl-1H-imidazo [4,5-b]pyrazi n-6-yl)-6-methyl-1H-pyrrolo[ 2,3-c] pyridin-7(6H)-one | 407.1 |
| 15 | 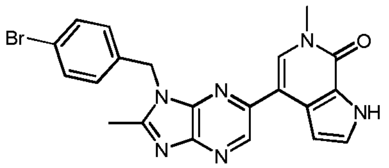 | 4-(1-(4-bromobenzyl)-2-meth yl-1H-imidazo[4,5-b]pyrazin-6-yl)-6-methyl-1H-pyrrolo[2, 3-c] pyridin-7(6H)-one | 449.06 |
| 16 | 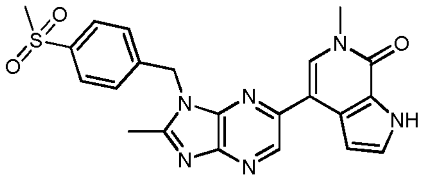 | 6-methyl-4-(2-methyl-1-(4-( methylsulfonyl) benzyl)-1H-i midazo[4,5-b]pyrazin-6-yl)-1 H-pyrrolo[2,3-c]pyridin-7(6H )-one | 449.1 |

**Example 2**

The effects of compound 1 on proliferation of human myeloproliferative tumor cells HEL and SET-2 cells with JAK2V617F activation mutation were tested using CTG assay in this example to evaluate half inhibitory rate ($IC_{50}$) of compound 1 on HEL and SET-2 cell proliferation.

**Method:**

1. Cell culture:

a) HEL cells were cultured in RPMI 1640 medium containing 1% PS and 10% FBS. The cells were incubated in a humidified incubator with 5% carbon dioxide ($CO_2$) at 37 °C.

b) SET-2 cells were cultured in RPMI 1640 medium containing 1% PS and 20% heat-inactive FBS. The cells were incubated in a humidified incubator with 5% carbon dioxide ($CO_2$) at 37 °C.

2. Cell plating:

a) Cells were harvested upon reaching a certain density.

b) Cells were resuspended in corresponding medium with a cerain density.

c) 30 μL of cell suspension was added into each well of a 384-well plate. For HEL, the density was 1800 cells/well, and for SET-2, 2000 cells/well.

d) Cells were incubated overnight in a humidified incubator with 5% $CO_2$ at 37 °C.

3. Compound Preparation

The compounds were diluted with DMSO 6.67 fold from 20 mM to 3 mM, then 3-fold serial dilutions were

conducted to make 10 concentrations for each compound. Staurosporine was diluted with DMSO 6.67 fold from 20 mM to 3 mM, followed by 3-fold serial dilutions to make 10 concentrations. The vehicle control consisted of 100% DMSO, and the positive control was 3 mM Staurosporine.

4. Compound Treatment

a) 24 hours post cell seeding, 100 nL of diluted compound from step 3 was added to each well of the 384-well plates.

b) The final concentrations of 2 compounds and Staurosporine were : 10000, 3333, 1111, 370.4, 123.5, 41.2, 13.7, 4.6, 1.5, 0.5 nM.

c) The positive control had a final concentration of 10 μM Staurosporine, and the vehicle control had a final concentration of 0.33% DMSO.

d) Cells were incubated for 72 h in a 37 °C, 5% $CO_2$ incubator.

5. CTG Detection

a) 30 μL CTG reagent (CelltiterGlo kit) was added to each well, and the plate was subjected to orbital shaking for 2 minutes, then incubated at room temperature in the dark for 30 minutes.

b) Luminescence values were obtained using Envision.

6. Data analysis

$IC_{50}$ (the half maximal inhibitory concentration) was calculated with GraphPad Prism 8 software and following nonlinear fitting formula:

$$Y=Bottom + (Top-Bottom)/(1+10^\wedge((LogIC_{50}-X)*HillSlope))$$

X: Log value of Compound concentration, Y : percentage inhibition rate (%inhibition)

$$\% \ Inhibition=(LUM_{High}-LUM_{cmpd})/ (LUM_{High}-LUM_{Low})*100$$

$LUM_{High}$ is the average value of 0.33% DMSO wells, $LUM_{cmpd}$ is the value of each well of the compound, $LUM_{Low}$ is the average value of 10 μM Staurosporine wells.

**Results:**

The proliferation inhibition curve of compound 1 against HEL cells at different concentrations is shown in Figure 1, and the proliferation inhibition curve against SET-2 cells is shown in Figure 2.

The $IC_{50}$ values of compound 1 against HEL cells and SET-2 cells are 154.1 nM and 103.6 nM, respectively. These results indicate that compound 1 exhibits a good inhibitory effect on the proliferation of both HEL and SET-2 cells.

Table 2. $IC_{50}$ (nM) for cell proliferation inhibition

| **Cell lines** | **Compound** | $IC_{50}$(nM) | Hillslope | Bottom | Top |
|---|---|---|---|---|---|
| HEL | Compound 1 | 154.1 | 1.8 | 6.4 | 102.5 |
| SET-2 | Compound 1 | 103.6 | 1.6 | 4.7 | 104.6 |

## Example 3

In present example, reporter gene assay was used to determine the inhibitory effect of compound 1 on NF-κB transcriptional activity in HEK293 cells, to evaluate the effect of the compound on NF-κB transcriptional activity.

**Method:**

1. Compound preparation

a) All compounds were 3-fold serial diluted from 10 mM for 10 doses in DMSO..

b) The positive compound CDDO-Me was 3-fold serial diluted from 10 mM for 10 doses in DMSO.

c) 1000 × positive control (100% DMSO) and 1000 × negative control (100% DMSO)were prepared.

d) Compounds plate was sealed and shaked for 5 minutes.

2. Cell suspension preparation

a) All cells were cultured following ATCC recommendations. HEK293T-NPκB cells were assayed during exponential growth phase.

b) The culture medium was removed from the flask, and then the cells were rinsed with PBS.

c) The cells were digested with trypsin. Digestion was terminated with complete culture medium, and the cells were collected and counted.

d) A total of $6*10^6$ HEK293T cells were seeded into a 100 mm cell culture dish.

e) The cells were then incubated overnight in a 5% $CO_2$ incubator.

3. Compound treatment

a) 25 nL diluted compounds from Step 1 were transferred to the cell culture plate with Echo 655.

b) Cells (prepared in step2) were seeded into the 384-well assay plate at a density of 17,000 cells/well. The seeding medium used was phenol red-free DMEM containing 5% charcoal-stripped FBS and 8 ng/ml TNFα.

c) The cells were incubated for 16-20 h in a 5% COzincubator at 37 °C.

4. Compound detection

a) The Britelite plus reagent were allowedto reach room temperature.

b) The 384-well assay plates (prepared at Step3) were allowed to reach room temperature.

c) 25 μL Britelite plus reagent was added to each well of 384-well assay plate.

d) Luminescence values were recorded with Envision.

5. Data analysis

5.1 The percent inhibition was calculated as follows:

$$\%Inhibition = 100 - \left[\frac{RLU_{Compound} - \overline{RLU}_{Positive}}{\overline{RLU}_{Vehicle} \quad \overline{RLU}_{Positive}}\right] * 100$$

RLU: relative luminescence units

$RLU_{Positive}$ : The average luminescence value of the blank controls.

$RLU_{vehicle}$ : The average luminescence value of the negative controls.

5.2 $IC_{50}$ calculating and compound dose-response curves fitting:

The following nonlinear fitting formula was used to calculate the $IC_{50}$ of the compound with Graphpad8.0.

$$Y=Bottom + (Top-Bottom)/(1+10^{\wedge}((LogIC_{50}-X)*HillSlope))$$

X: log value of compound concentration; Y: Compound percentage inhibition

**Results:**

The inhibition curve of compound 1 on NF-κB transcriptional activity at different concentrations was shown in Figure 3.

Under the conditions of this experiment, compound 1 significantly inhibited the transcriptional activity of NF-κB in HEK293 cells stably transfected with the NF-κB reporter gene.

Table 3. $IC_{50}$ (nM) for the inhibition of NF-κB transcriptional activity

| Compound | $IC_{50}$(nM) | Hillslope | Bottom | Top |
|---|---|---|---|---|
| Compound 1 | 147.6 | 1.3 | -6.5 | 87.4 |

**Example 4**

The Human IL-6 Valukine ELISA kit (purchased from R&D) was used to measure the IL-6 content in the cell supernatants by ELISA method in this example. The experimental procedures shown below were conducted to determine the effect of compound 1 on IL-6 secretion in THP-1 cell supernatants following LPS stimulation.

**Method:**

1. Cell culture:
THP-1 cells were cultured in RPMI 1640 medium containing 1% PS and 10% FBS and 2-Mercaptoethanol (1X). The cells were incubated in a humidified incubator with 5% carbon dioxide ($CO_2$) at 37 °C.

2. Cell plating:

   a) The cells were harvested when the confluence reached 80%-90%.

   b) Subsequently, the cells were resuspended to a certain density with RPMI Medium 1640 containing 1% PS

and 10% heat-inactive FBS.

c) The cell suspension was added to a 96-well plate, 100 μL per well, and the cell density was 150,000/well.

d) The cells were incubated in a humidified incubator with 5% $CO_2$ at 37 °C.

3. Compound preparation

a) Compound 1 was diluted 2 fold with DMSO from 20 mM to 10 mM, followed by further 3-fold serial dilutions to generate 9 concentrations.

b) Vehicle control was 100% DMSO.

c) A working stock solution was made by adding 5 μL of the serial-diluted compound to 45 μL culture medium.

4. Compound treatment

a) 96 μL mediun and 2 μL compound working stock solution from Step 3 were added to each well.

b) After 30 minutes incubation, 2 μL LPS was adde to each well, resulting in a final concentration of 4 μg/mL.

c) The final testing concentrations of compound 1 were: 10000, 3333, 1111, 370.4, 123.5, 41.2, 13.7, 4.6, 1.5, 0nM.

d) The final vehicle control comprised 0.1% DMSO, the blank control was cells without LPS treatment.

e) The cells were incubated in a 5% COzincubator at 37 °C.

5. IL-6 ELISA Detection

a) 96-well plate was centrifuged for 5 minutes at 1500 rpm, and pipetted 150 μL/well cellular supernatant from each well.

b) Before proceeding, all reagents were left to equilibrate to room temperature and mixed thoroughly to avoid foaming.

c) Determine the number of strips required based on the number of experimental wells (blanks and standards).

d) Add samples: 100 μL diluted standards and test samples were added to each well, sealed and incubated for 2 hours at room temperature.

e) Washing: The liquid in the well was removed, 300 μL 1× Wash Buffer was added to each well; discarded the liquid in the well after staying for 1 minute. Repeated 3 times, dried it on filter paper each time.

f) Add detection antibody: 200 μL Human IL-6 Conjugate was added to each well, thensealed and incubated for 2 hours at room temperature.

g) Washing: Repeated Step e).

h) Reaction: 200 μL Substrate Solution was added to each well, incubated for 20 minues at room temperature, in dark conditions.

i) Termination reaction: 50 μL Stop solution was added to each well quickly to terminate the reaction.

j) Plate reading: Within 30 minutes after termination, read the value using the measurement wavelength 450 nm.

6. CTG Detection

a) 50 μL medium was added to each well.

b) 100 μL CTG reagent (CelltiterGlo kit) was added to each well, and the plate was placed on a orbital shaker for 2 minutes, then placed at room temperature in dark conditions for 30 minutes, and then transferred to CTG plate.

c) The luminescence values were recorded with Envision.

7. Data analysis

$IC_{50}$ (The half maximal inhibitory concentration) was calculated using GraphPad Prism 8 software with the following nonlinear fitting formula:

$$Y=Bottom + (Top-Bottom)/(1+10^{\wedge}((LogIC_{50}-X)*HillSlope))$$

a) CTG detection: X: Log value of Compound concentration, Y : cell viability inhibition rate (Inhibition%)

$$Inhibition\ \%=(1-Compd/Vehicle\ control)*100$$

b) IL-6 detection: X: Log value of Compound concentration, Y : IL-6 concentration (pg/mL)

IL-6 concentration was calculated based on the linear equation of standard dilution concentration and wavelength detection.
Plate 1: y=0.0117x+0.0421 $R^2$=0.9998 Plate 2: y=0.0111x+0.0445 $R^2$=0.9996

Note: When the cell viability inhibition rate is greater than 30%, IL-6 at this concentration dose not participate in the calculation of $IC_{50}$.

**Results:**

The inhibitory curve of compound 1 on IL-6 in THP-1 cells at different concentrations was shown in Figure 4.

As compound concentration increased, the concentration of IL-6 in the supernatant of THP-1 cells decreased, indicating that compound 1 significantlyinhibited the IL-6 secretion in THP1 monocytes.

Table 4. $IC_{50}$ (nM) for inhibition of IL-6 secretion in THP-1 cells

| Compound | $IC_{50}$(nM) | Hillslope | Bottom | Top |
|---|---|---|---|---|
| Compound 1 | 22.4 | -2.7 | 4.2 | 53.9 |

The above data demonstrated that the compound of the present invention displayed a significant inhibitory effect on the proliferation of human myeloproliferative tumor cell lines HEL and SET-2 harboring the JAK2V617F mutantion, which is directly associated with myelofibrosis. Furthermore, the compound exhibited a substantial inhibitory effect on the transcriptional activity of NF-κB and the secretion of the inflammatory factor IL-6.

**Example 5**

A Phase I study to evaluate safety, tolerability, pharmacokinetics and efficacy of compound 1 and determine MTD and RP2D

**Target:** Subjects with primary myelofibrosis, post-polycythemia vera myelofibrosis, post-essential thrombocythemia myelofibrosis.

**Inclusion Criteria:**

1. Male or female adults, aged ≥ 18 years;
2. Subjects have diagnosis of primary myelofibrosis (PMF) according to WHO criteria or or diagnosis of post-polycythemia vera myelofibrosis (post-PV MF) or post-essential thrombocythemia myelofibrosis (post-ET MF) according to IWG-MRT criteria;
3. Dose escalation phase: Myelofibrosis (MF) patients have "intermediate-1 risk" and above according to Dynamic International Prognostic Scoring System (DIPSS) (with one or more adverse prognostic factors) and have received at least one therapy, patients cannot benefit from existing therapy or the patient has been assessed by the Investigator as not being a suitable candidate or otherwise ineligible for the standard of care therapy.
4. Dose expansion phase: Myelofibrosis (MF) patients have "intermediate-1 risk" and above according to Dynamic International Prognostic Scoring System (DIPSS) (with one or more adverse prognostic factors);
5. The expanded enrollment study requires patients with symptomatic splenomegaly: Palpate the edge of the spleen to reach or exceed at least 5 cm below the ribs (the distance from the rib edge to the farthest point of the spleen);
6. Expected survival ≥ 24 weeks months;
7. Eastern Cooperative Oncology Group (ECOG) performance status 0-2;
8. Summation of all the individual scores ≥1, evaluated by the MPN Symptoms Assessment Form(MPN-SAF);
9. Bone marrow and peripheral blood blast count≤20%;
10. Patients who have not had splenic radiotherapy within 6 months before screening;
11. Adequate organ function defined as:

    a) Blood routine: Absolute neutrophil count (ANC) ≥ $1.0\times10^9$/L, platelets (PLT) ≥ 100 $\times$ $10^9$/L, hemoglobin (HGB) ≥ 7.5 g/dL (75 g/L ) (No red blood cell transfusions within 14 days before the screening test);
    b) Coagulation function: Subjects who are not receiving anticoagulation therapy, international normalized ratio (INR) and activated partial thromboplastin time (APTT) ≤ 1.5 times the upper limit of normal values(ULN);
    c) Liver: Serum total bilirubin (TBIL) ≤2.0 × ULN, if the subjects have Gilbert syndrome, TBIL is required to be ≤3.0 × ULN; aspartate aminotransferase (AST) and alanine aminotransferase (ALT) ≤2.5 × ULN;
    d) Kidneys: Serum creatinine (Scr) ≤1.5 × ULN or creatinine clearance (Ccr) ≥ 50 mL/min (calculated by Cockcroft-Gault formula);

12. The toxic reactions of previous anti-tumor treatments have returned to the severity before treatment or NCI CTCAE version 4.03 ≤ grade 1 (the same is true for alopecia or other toxicities that are considered to have no safety risk to the patient in neonates);
13. Women of child-bearing potentiall must be non-lactating period, and the result of a serum pregnancy test performed within 7 days before starting treatment must be negative; all subjects must use medically approved contraceptive measures during the entire treatment and within 3 months after the last dose of study drug;
14. Subjects must comply with the study protocol and and follow-up procedures;
15. Subjects must have signed written, informed consent.

**Dose Escalation Study Design:**

This study used a "3+3" design and designed 3 dose levels initially, that is, after determining the initial dose of 60 mg, the test doses were increased by 33% and 25%, which were 60 mg/d, 80 mg/d and 100 mg/d respectively. "3+3" design escalation rule: ① If no DLTs are observed of 3 DLT-evaluable patients, the trial proceeds to next higher dose cohort; (2)If one subject develops a DLT of 3 DLT-evaluable patients, an additional three subjects are enrolled into that same dose cohort, once ≥ 1 DLTs are observed further (total number of DLT patients≥2), the maximum tolerated dose (MTD) will be declared as the previous dose level; ③ If 2 or more patients in a cohort experience DLTs, then stop enrollment at the specific dose and higher dose levels, the MTD will be declared as the previous dose level.

Twice daily (BID, Q12H was used in this study, two doses per day every 12 hours), then the dose level and dosing regimens (including, but not limited to, starting dose, intended escalating maximum dose, intermediate dose, dosing frequency and intervals) can be adjusted by the sponsor and researcher based on the result of pharmacokinetics, safety, tolerability and efficacy.21 days each treatment cycle.

**Efficacy evaluation method:**

a) Routine blood test and peripheral blood smear

b) Bone marrow biopsy or bone marrow cytology smear

c) MRI

d) Palpation of spleen

e) Spleen ultrasound

f) Evaluation of myelofibrosis degree

g) Evaluation of bone marrow hyperplasia grade

**Efficacy evaluation criteria:**

The 2013 ELN and IWG-MRT consensus standards were adopted.

Table 5. Efficacy evaluation criteria

| Response categories | Required criteria |
|---|---|
| Complete Remission (CR) | All the following categories must be qualified:<br>(1) Bone marrow: Age-adjusted normocellularity; <5% blasts; ≤grade 1 MF(European classification);<br>(2) Peripheral blood: HGB≥100 g/L, ANC≥1×$10^9$/L; PLT≥100×$10^9$/L, none of the above indicators have reached upper normal limit; <2% immature myeloid cells;<br>(3) Clinical: Resolution of disease symptoms; spleen and liver not palpable; no evidence of extramedullary hematopoiesis. |
| Partial Remission (PR ) | Belong to one of the following categories:<br>(1) Peripheral blood: HGB≥100 g/L, ANC≥1×$10^9$/L, PLT≥100×$10^9$/L, none of the above indicators have reached upper normal limit; <2% immature myeloid cells; Clinical: Resolution of disease symptoms; spleen and liver not palpable; no evidence of extramedullary hematopoiesis.<br>(2) Bone marrow: Age-adjusted normocellularity; <5% blasts; ≤grade 1 MF and peripheral blood: HGB (85~< 100) g/L, ANC≥1×$10^9$/L, PLT (50-< 100)×$10^9$/L, none of the above indicators have reached upper normal limit; <2% immature myeloid cells; Clinical: Resolution of disease symptoms; spleen and liver not palpable; complete resolution of physical signs, including hepatosplenomegaly; no evidence of extramedullary hematopoiesis. |
| Clinical improvement(CI) | The achievement of anemia, splenomegaly or symptoms response without progressive disease increase in severity of anemia, thrombocytopenia, or neutropenia.<br>Anemia response: Transfusion-independent patients: a ≥20 g/L increase in hemoglobin level; Transfusion-dependent patients: becoming transfusion-independent (absence of red blood cells transfusions during the treatment phase and HGB≥85 g/L).<br>Spleen response:(1) A baseline splenomegaly at 5-10 cm below the left costal margin becomes not palpable;(2) A baseline splenomegaly that is palpable at >10 cm, below the left costal margin, decreases by ≥ 50%;(3) A baseline splenomegaly that is palpable at <5 cm, below the left costal margin, is not eligible for spleen response;(4) A spleen response requires confirmation by MRI or computed tomography showing ≥35% spleen volume reduction.<br>Symptoms response : A >50% reduction in the MPN-SAF TSS |

(continued)

| Response categories | Required criteria |
|---|---|
| Progressive disease(PD)(PD) | Belong to one of the following categories:<br>(1) Appearance of a new splenomegaly that is palpable at least 5 cm below the left costal margin;<br>(2) A ≥ 100% increase in palpable distance, below left costal margin, for baseline splenomegaly of 5-10 cm;<br>(3) A >50% increase in palpable distance, below left costal margin, for baseline splenomegaly of >10 cm; (4) Leukemic transformation confirmed by a bone marrow blast count of ≥20%;<br>(5) A peripheral blood blast content of ≥20% associated with an absolute blast count of A 1 × 10(9)/L that lasts for at least 2 weeks. |
| Stable disease (SD) | Belonging to none of the above listed response categories. |
| Relapse | Belong to one of the following categories:<br>(1) No longer meeting criteria for at least CI after achieving CR, PR, or CI;<br>(2) Loss of anemia response persisting for at least 1 month;<br>(3) Loss of spleen response persisting for at least 1 month. |
| Cytogenetic remission | At least 10 metaphases must be analyzed for cytogenetic response evaluation and requires confirmation by repeat testing within 6 months window.<br>(1) CR: Eradication of a preexisting cytogenetic abnormalities;<br>(2) PR: X50% reduction in preexisting abnormal metaphases (partial response applies only to patients with at least ten abnormal metaphases at baseline). |
| Molecular remission | Molecular response evaluation must be analyzed in peripheral blood granulocytes and requires confirmation by repeat testing within 6 months window.<br>(1) CR: Eradication of a preexisting molecular biology;<br>(2) PR: X50% decrease in allele burden(partial response applies only to patients with at least 20% mutant allele burden at baseline) . |
| Cytogenetic/ molecular relapse | Re-emergence of a pre-existing cytogenetic or molecular abnormality that is confirmed by repeat testing |

The above descriptions are only preferred embodiments of the present invention, and are not intended to limit the present invention. For those skilled in the art, the present invention may have various modifications and changes.. Any modifications, equivalent substitutions, improvements, etc. made within the spirit and principles of the present invention shall be included in the protection scope of the present invention.

## Claims

**1.** Use of a compound in the preparation of a medicament for treating myelofibrosis and the symptoms/signs associated with myelofibrosis, wherein the compound is selected from one or more of the compound of formula I and the pharmaceutically acceptable salts, prodrugs, solvates and hydrates thereof:

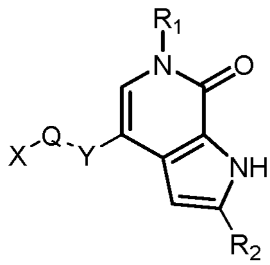

formula I

in the formula I,

$R_1$ and $R_2$ are each independently selected from H, $C_{1-6}$ alkyl, $C_{1-6}$ alkoxy, $C_{6-10}$ aryl or $C_{5-10}$ heteroaryl, wherein the $C_{1-6}$ alkyl, $C_{1-6}$ alkoxy, $C_{6-10}$ aryl or $C_{5-10}$ heteroaryl is optionally substituted with $C_{1-6}$ alkyl group, $-NH_2$ group, -OH group, $C_{6-10}$ aryl group or $C_{5-10}$ heteroaryl group; and the $C_{5-10}$ heteroaryl and the $C_{5-10}$ heteroaryl group has 1, 2, or 3 heteroatoms independently selected from nitrogen, oxygen or sulfur;
Q is absent or selected from $C_{1-6}$ alkylene, $-SO_2-$ or -NH-, wherein the $C_{1-6}$ alkylene or -NH- is optionally substituted with halogen, $C_{1-6}$ alkyl or $C_{1-6}$ alkoxy;
X is selected from H, $C_{1-6}$ alkyl, $C_{6-10}$ aryl or $C_{5-10}$ heteroaryl, wherein the $C_{1-6}$ alkyl, $C_{6-10}$ aryl or $C_{5-10}$ heteroaryl is optionally substituted by halogen, halo $C_{1-6}$ alkyl, $C_{1-6}$ alkyl, $C_{1-6}$ alkoxy, $C_{1-6}$ alkylthio, $C_{1-6}$ alkoxycarbonyl or $C_{1-6}$ alkyl-$SO_2$-;
Y is

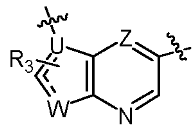,

wherein, - - - represents a single bond or a double bond, the U, W or Z is independently selected from C or N;
$R_3$ is absent or selected from H, halogen, hydroxyl, amino, $C_{1-6}$ alkyl, $C_{1-6}$ alkoxy, cyano, oxo, or $-N(R_4)-SO_2-R_5$;
$R_4$ and $R_5$ are each independently selected from H, $C_{1-6}$ alkyl or halo $C_{1-6}$ alkyl.

2. The use of claim 1, wherein, Y is

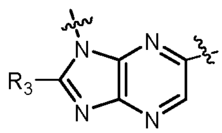.

3. The use of claim 1 or 2, wherein,

$R_1$ is selected from H or $C_{1-4}$ alkyl, preferably, $R_1$ is H, methyl, ethyl, propyl, isopropyl, n-butyl, sec-butyl, tert-butyl; and/or
$R_2$ is selected from H or $C_{1-3}$ alkyl; preferably, $R_2$ is H, methyl, ethyl, propyl, isopropyl; and/or
Q is absent or $C_{1-3}$ alkylene; preferably, Q is selected from methylene or ethylene; and/or
X is selected from H, $C_{1-3}$ alkyl or phenyl, alternatively, phenyl can be substituted with halogen, halo $C_{1-3}$ alkyl, $C_{1-3}$ alkyl, $C_{1-3}$ alkoxy or $C_{1-3}$ alkyl-$SO_2$-.

4. The use of claim 1, wherein, the compound is selected from one or more of the compound of formula II and the pharmaceutically acceptable salts, prodrugs, solvates and hydrates thereof:

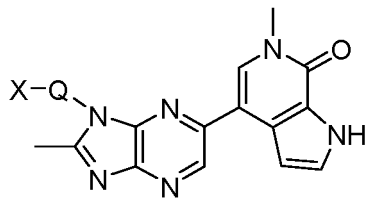

formula II

in the formula II,

Q is $C_{1-6}$ alkylene;
X is phenyl, the phenyl is optionally substituted with halogen, halo $C_{1-3}$ alkyl, $C_{1-3}$ alkyl, $C_{1-3}$ alkoxy or $C_{1-3}$ alkyl-$SO_2$-.

**5.** The use of claim1, wherein the compound is selected form one or more of:

6-methyl-4-(2-methyl-1-(4-(trifluoromethyl)benzyl)-1H-imidazo[4,5-b]pyrazin-6-yl)-1H-pyrrolo[2,3-c]pyridin-7(6H)-one;
4-(1-(4-chlorobenzyl)-2-methyl-1H-imidazo[4,5-b]pyrazin-6-yl)-6-methyl-1H-pyr rolo[2,3-c]pyridin-7(6H)-one;
4-(1-(4-methoxybenzyl)-2-methyl-1H-imidazo[4,5-b]pyrazin-6-yl)-6-methyl-1H-p yrrolo[2,3-c]pyridin-7(6H)-one;
4-(1-benzyl-2-methyl-1H-imidazo[4,5-b]pyrazin-6-yl)-6-methyl-1H-pyrrolo[2,3-c ]pyridin-7(6H)-one;
4-(1-(3-trifluoromethylbenzyl)-2-methyl-1H-imidazo[4,5-b]pyrazin-6-yl)-6-methy 1-1H-pyrrolo[2,3-c]pyridin-7(6H)-one;
4-(1-(2-fluoro-5-trifluoromethylbenzyl)-2-methyl-1H-imidazo[4,5-b]pyrazin-6-yl) -6-methyl-1H-pyrrolo[2,3-c]pyridin-7(6H)-one;
4-(1-(3-fluoro-5-trifluoromethylbenzyl)-2-methyl-1H-imidazo[4,5-b]pyrazin-6-yl) -6-methyl-1H-pyrrolo[2,3-c]pyridin-7(6H)-one;
4-(1-(2-fluoro-4-chlorobenzyl)-2-methyl-1H-imidazo[4,5-b]pyrazin-6-yl)-6-methy 1-1H-pyrrolo[2,3-c]pyridin-7(6H)-one;
4-(1-(3-trifluoromethyl-4-chlorobenzyl)-2-methyl-1H-imidazo[4,5-b]pyrazin-6-yl) -6-methyl-1H-pyrrolo[2,3-c]pyridin-7(6H)-one;
4-(1-(3-chloro-4-trifluoromethylbenzyl)-2-methyl-1H-imidazo[4,5-b]pyrazin-6-yl) -6-methyl-1H-pyrrolo[2,3-c]pyridin-7(6H)-one;
4-(1-(3-chlorobenzyl)-2-methyl-1H-imidazo[4,5-b]pyrazin-6-yl)-6-methyl-1H-pyr rolo[2,3-c]pyridin-7(6H)-one;
4-(1-(2,4-difluorobenzyl)-2-methyl-1H-imidazo[4,5-b]pyrazin-6-yl)-6-methyl-1H-pyrrolo[2,3-c]pyridin-7(6H)-one;
4-(1-(4-bromobenzyl)-2-methyl-1H-imidazo[4,5-b]pyrazin-6-yl)-6-methyl-1H-pyr rolo[2,3-c]pyridin-7(6H)-one;
6-methyl-4-(2-methyl-1-(4-(methylsulfonyl)benzyl)-1H-imidazo[4,5-b]pyrazin-6-yl)-1H-pyrrolo[2,3-c]pyridin-7(6H)-one;
preferably, the compound is 6-methyl-4-(2-methyl-1-(4-(trifluoromethyl)benzyl)-1H-imidazo[4,5-b]pyrazin-6-yl)-1 H-pyrrolo[2,3-c]pyridin-7(6H)-one.

**6.** The use of any one of the claims 1-5, wherein the medicament also comprises excipients.

**7.** The use of any one of the claims 1-5, wherein the medicament is administered intravenously, intramuscularly, parenterally, nasally, orally or rectally.

**8.** A method of treating myelofibrosis and the symptoms/signs associated with myelofibrosis, wherein the method comprising:
providing a medicament comprising the compound, wherein the compound is selected from one or more of the compound of formula I and the pharmaceutically acceptable salts, prodrugs, solvates and hydrates thereof; and administering therapeutically effective amount of the medicament to subject having myelofibrosis;

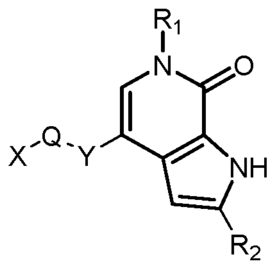

formula I

in the formula I,

$R_1$ and $R_2$ are each independently selected from H, $C_{1-6}$ alkyl, $C_{1-6}$ alkoxy, $C_{6-10}$ aryl or $C_{5-10}$ heteroaryl, wherein the $C_{1-6}$ alkyl, $C_{1-6}$ alkoxy, $C_{6-10}$ aryl or $C_{5-10}$ heteroaryl is optionally substituted with $C_{1-6}$ alkyl group, $-NH_2$ group, -OH group, $C_{6-10}$ aryl group or $C_{5-10}$ heteroaryl group; and the $C_{5-10}$ heteroaryl and the $C_{5-10}$ heteroaryl group has 1, 2, or 3 heteroatoms independently selected from nitrogen, oxygen or sulfur;
Q is absent or selected from $C_{1-6}$ alkylene, $-SO_2-$ or -NH-, wherein the $C_{1-6}$ alkylene or -NH- is optionally substituted with halogen, $C_{1-6}$ alkyl or $C_{1-6}$ alkoxy;
X is selected from H, $C_{1-6}$ alkyl, $C_{6-10}$ aryl or $C_{5-10}$ heteroaryl, wherein the $C_{1-6}$ alkyl, $C_{6-10}$ aryl or $C_{5-10}$ heteroaryl is optionally substituted by halogen, halo $C_{1-6}$ alkyl, $C_{1-6}$ alkyl, $C_{1-6}$ alkoxy, $C_{1-6}$ alkylthio, $C_{1-6}$ alkoxycarbonyl or $C_{1-6}$ alkyl-$SO_2$-;
Y is

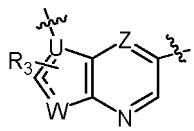 ,

wherein, - - - represents a single bond or a double bond, the U, W or Z is independently selected from C or N; $R_3$ is absent or selected from H, halogen, hydroxyl, amino, $C_{1-6}$ alkyl, $C_{1-6}$ alkoxy, cyano, oxo, or $-N(R_4)-SO_2-R_5$; $R_4$ and $R_5$ are each independently selected from H, $C_{1-6}$ alkyl or halo $C_{1-6}$ alkyl.

**9.** The method of claim 8, wherein, Y is

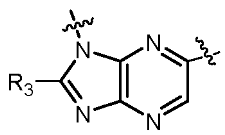 .

**10.** The method of claim 8, wherein,

$R_1$ is selected from H or $C_{1-4}$ alkyl, preferably, $R_1$ is H, methyl, ethyl, propyl, isopropyl, n-butyl, sec-butyl, tert-butyl; and/or
$R_2$ is selected from H or $C_{1-3}$ alkyl; preferably, $R_2$ is H, methyl, ethyl, propyl, isopropyl; and/or
Q is absent or $C_{1-3}$ alkylene; preferably, Q is selected from methylene or ethylene; and/or
X is selected from H, $C_{1-3}$ alkyl or phenyl, alternatively, phenyl can be substituted with halogen, halo $C_{1-3}$ alkyl, $C_{1-3}$ alkyl, $C_{1-3}$ alkoxy or $C_{1-3}$ alkyl-$SO_2$-.

**11.** The method of claim 8, wherein, the compound is selected from one or more of the compound of formula II and the pharmaceutically acceptable salts, prodrugs, solvates and hydrates thereof:

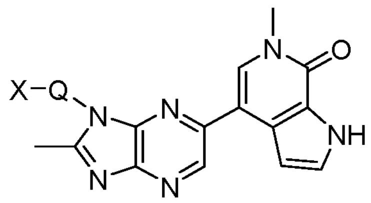

formula II

in the formula II,

Q is $C_{1-6}$ alkylene;
X is phenyl, the phenyl is optionally substituted with halogen, halo $C_{1-3}$ alkyl, $C_{1-3}$ alkyl, $C_{1-3}$ alkoxy or $C_{1-3}$ alkyl-$SO_2$-.

**12.** The method of claim 8, wherein, the compound is selected form one or more of:

6-methyl-4-(2-methyl-1-(4-(trifluoromethyl)benzyl)-1H-imidazo[4,5-b]pyrazin-6-yl)-1H-pyrrolo[2,3-c]pyridin-7(6H)-one;
4-(1-(4-chlorobenzyl)-2-methyl-1H-imidazo[4,5-b]pyrazin-6-yl)-6-methyl-1H-pyr rolo[2,3-c]pyridin-7(6H)-one;
4-(1-(4-methoxybenzyl)-2-methyl-1H-imidazo[4,5-b]pyrazin-6-yl)-6-methyl-1H-p yrrolo[2,3-c]pyridin-7(6H)-one;
4-(1-(4-methoxybenzyl)-2-methyl-1H-imidazo[4,5-b]pyrazin-6-yl)-6-methyl-1H-p yrrolo[2,3-c]pyridin-7(6H)-one;
4-(1-benzyl-2-methyl-1H-imidazo[4,5-b]pyrazin-6-yl)-6-methyl-1H-pyrrolo[2,3-c ]pyridin-7(6H)-one;
4-(1-(3-trifluoromethylbenzyl)-2-methyl-1H-imidazo[4,5-b]pyrazin-6-yl)-6-methy 1-1H-pyrrolo[2,3-c]pyridin-7(6H)-one;
4-(1-(2-fluoro-5-trifluoromethylbenzyl)-2-methyl-1H-imidazo[4,5-b]pyrazin-6-yl) -6-methyl-1H-pyrrolo[2,3-c]pyridin-7(6H)-one;
4-(1-(3-fluoro-5-trifluoromethylbenzyl)-2-methyl-1H-imidazo[4,5-b]pyrazin-6-yl) -6-methyl-1H-pyrrolo[2,3-c]pyridin-7(6H)-one;
4-(1-(2-fluoro-4-chlorobenzyl)-2-methyl-1H-imidazo[4,5-b]pyrazin-6-yl)-6-methy 1-1H-pyrrolo[2,3-c]pyridin-7(6H)-one;
4-(1-(3-trifluoromethyl-4-chlorobenzyl)-2-methyl-1H-imidazo[4,5-b]pyrazin-6-yl) -6-methyl-1H-pyrrolo[2,3-c]pyridin-7(6H)-one;
4-(1-(3-fluoro-4-trifluoromethylbenzyl)-2-methyl-1H-imidazo[4,5-b]pyrazin-6-yl) -6-methyl-1H-pyrrolo[2,3-c]pyridin-7(6H)-one;
4-(1-(3-chloro-4-trifluoromethylbenzyl)-2-methyl-1H-imidazo[4,5-b]pyrazin-6-yl) -6-methyl-1H-pyrrolo[2,3-c]pyridin-7(6H)-one;
4-(1-(3-chlorobenzyl)-2-methyl-1H-imidazo[4,5-b]pyrazin-6-yl)-6-methyl-1H-pyr rolo[2,3-c]pyridin-7(6H)-one;
4-(1-(2,4-difluorobenzyl)-2-methyl-1H-imidazo[4,5-b]pyrazin-6-yl)-6-methyl-1H-pyrrolo[2,3-c]pyridin-7(6H)-one;
4-(1-(4-bromobenzyl)-2-methyl-1H-imidazo[4,5-b]pyrazin-6-yl)-6-methyl-1H-pyr rolo[2,3-c]pyridin-7(6H)-one;
6-methyl-4-(2-methyl-1-(4-(methylsulfonyl)benzyl)-1H-imidazo[4,5-b]pyrazin-6-yl)-1H-pyrrolo[2,3-c]pyridin-7(6H)-one;
preferably, the compound is 6-methyl-4-(2-methyl-1-(4-(trifluoromethyl)benzyl)-1H-imidazo[4,5-b]pyrazin-6-yl)-1 H-pyrrolo[2,3-c]pyridin-7(6H)-one.

**13.** The method of claim 8, wherein, the medicament also comprises excipients.

**14.** The method of claim 8, wherein, the medicament is administered intravenously, intramuscularly, parenterally, nasally, orally or rectally.

**15.** The method of claim 8, wherein the compound is administered in a dosage of 1~500mg/day, preferably, the compound is administered orally in a dosage of about 3mg/day, or 5mg/ day, or 10mg/ day, or 20mg/ day, or 25mg/ day, or 30mg/ day, or 35mg/ day, or 40mg/ day, or 45mg/ day, or 50mg/ day, or 55mg/ day, or 60mg/ day, or 70mg/ day, or 80mg/ day, or 90mg/ day, or 100mg/ day, or 150mg/ day, or 200mg/ day.

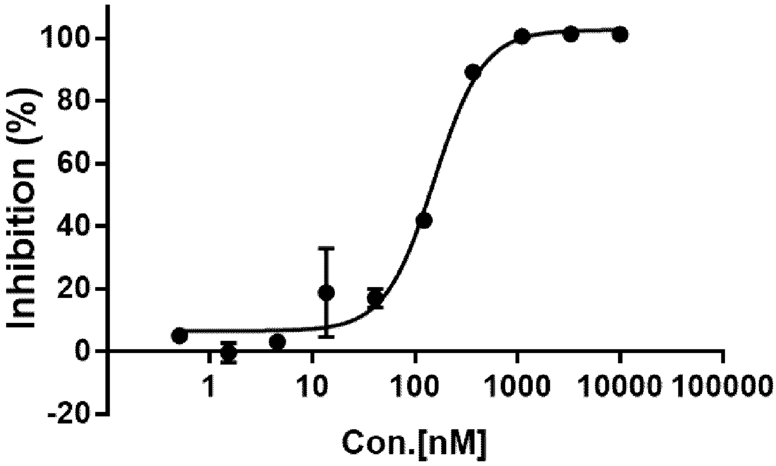


Figure 1

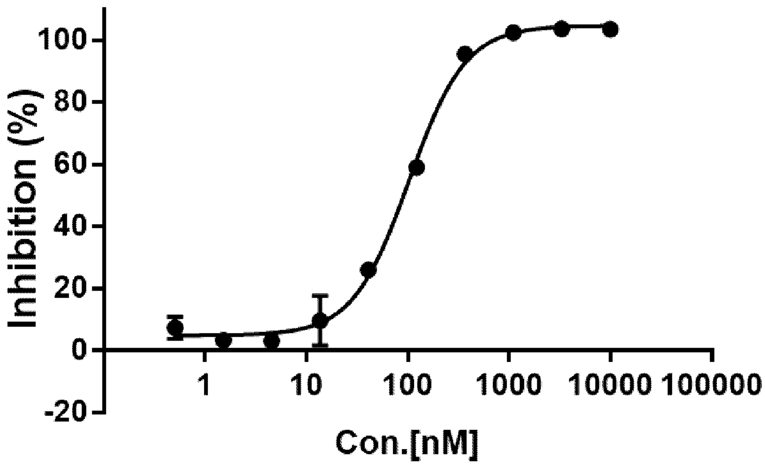


Figure 2

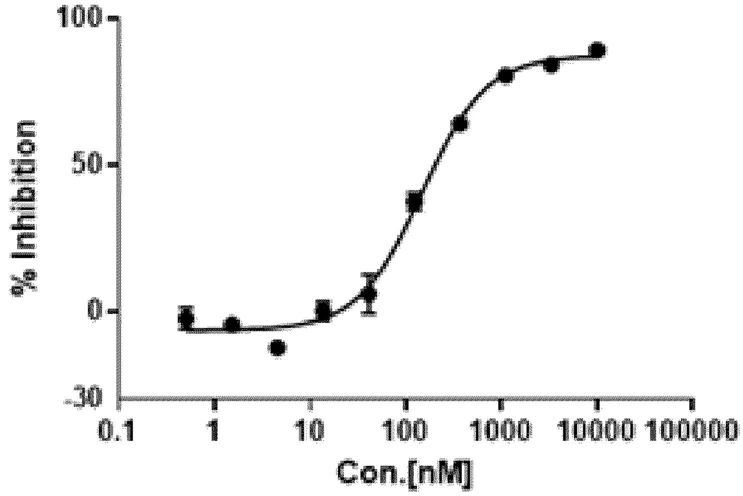


Figure 3

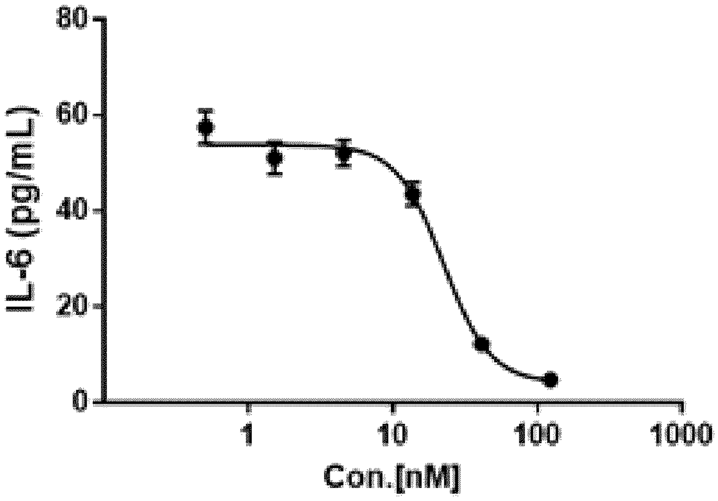


Figure 4

**INTERNATIONAL SEARCH REPORT**

International application No. **PCT/CN2022/100134**

**A. CLASSIFICATION OF SUBJECT MATTER**

A61K 31/4985(2006.01)i； A61P 35/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

DWPI, CNABS(CN), CNTXT, WOTXT, USTXT, EPTXT, CNKI(CN), PUBMED, CASLINK: 骨髓纤维化, Myelofibrosis, STN结构式检索 等, STN structural formula search et al.

**C. DOCUMENTS CONSIDERED TO BE RELEVANT**

| Category* | Citation of document, with indication, where appropriate, of the relevant passages | Relevant to claim No. |
|---|---|---|
| Y | WO 2019120234 A2 (BETTA PHARMACEUTICALS CO., LTD.) 27 June 2019 (2019-06-27)<br>entire document, in particular, claims 1-36, and description, p. 13, paragraph 2 to p. 15, paragraph 5, and embodiments 1-36 and 38 | 1-15 |
| Y | CN 111727189 A (THE BOARD OF TRUSTEES OF UNIVERSITY OF ILLINOIS) 29 September 2020 (2020-09-29)<br>entire document, in particular, claims 1-58, and description, paragraphs [0251] and [0572]-[0588] | 1-15 |
| Y | TREMBLAY, D. et al. . "Next Generation Therapeutics for the Treatment of Myelofibrosis" *Cells,* Vol. 10, No. 5, 27 April 2021 (2021-04-27),<br>1034; pp. 1-19, in particular, abstract, and section 3.2.1 | 1-15 |
| PY | WO 2021233371 A1 (BETTA PHARMACEUTICALS CO., LTD.) 25 November 2021 (2021-11-25)<br>entire document, in particular, claims 1, 31, and 35-46, and description, p. 17, paragraph 3 to p. 19, paragraph 4 | 1-15 |
| PY | US 2021379074 A1 (INCYTE CORP.) 09 December 2021 (2021-12-09)<br>description, paragraphs [0030]-[0031] and [0066]-[0067], and embodiments 1, 4, and 5 | 1-15 |

☐ Further documents are listed in the continuation of Box C.  ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 August 2022** | **22 September 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/100134**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **8-15**
because they relate to subject matter not required to be searched by this Authority, namely:

[1] A method for treating a human or animal body (PCT Rule 39.1(iv)).

[2] However, the examiner still conducted a search with respect to the assumption that the subject matter of claims 8-15 is amended to be a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/100134**

| Patent document cited in search report | Publication date (day/month/year) | Patent family member(s) | Publication date (day/month/year) |
|---|---|---|---|
| WO 2019120234 A2 | 27 June 2019 | US 2020385408 A1 | 10 December 2020 |
| | | CA 3086054 A1 | 27 June 2019 |
| | | KR 20200101434 A | 27 August 2020 |
| | | TW 201927792 A | 16 July 2019 |
| | | CN 111712496 A | 25 September 2020 |
| | | JP 2021506903 A | 22 February 2021 |
| | | AU 2018391833 A1 | 30 July 2020 |
| | | EP 3719017 A2 | 07 October 2020 |
| | | SG 11202005793S A | 29 July 2020 |
| | | BR 112020012527 A2 | 24 November 2020 |
| | | IL 275478 A | 31 August 2020 |
| CN 111727189 A | 29 September 2020 | EP 3717487 A1 | 07 October 2020 |
| | | WO 2019109057 A1 | 06 June 2019 |
| | | CA 3118400 A1 | 06 June 2019 |
| | | US 2021002272 A1 | 07 January 2021 |
| WO 2021233371 A1 | 25 November 2021 | None | |
| US 2021379074 A1 | 09 December 2021 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **KLAMPFL T et al.** *NEJM,* 2013, vol. 369 (25), 2379-90
- **NANGALIA J et al.** *NEJM,* 2013, vol. 369 (25), 2391-405
- **TEFFERI A.** *Am J Hematol.,* January 2021, vol. 96 (1), 145-162
- *Blood,* 2012, vol. 119 (12), 2721-2730